# EUROPEAN PATENT SPECIFICATION

(11) **EP 2 438 963 B1**
(45) Date of publication and mention of the grant of the patent: **04.01.2017**
(21) Application number: 11447024.8
(22) Date of filing: 05.10.2011
(51) Int. Cl.: A61Q 5/02, A61Q 19/10, A61K 8/34, A61K 8/41, A61K 8/42, C11D 1/52, C11D 1/65, C11D 1/835, C11D 1/86, C11D 1/94, C11D 3/20, C11D 3/30

(54) **Homogeneous monoalkanolamide blend**
Homogene Monoalkanolamidmischung
Mélange homogène de monoalkanolamide

(30) Priority: 06.10.2010 EP 10447019
(43) Date of publication of application: 11.04.2012
(73) Proprietor: EOC Surfactants N.V., 9940 Evergem (BE)
(72) Inventor: Van Haesebroeck, Nick, 9870 Zulte (BE); Nuyttens, Frank, 8893 St.Eloois Vijve (BE); Oudt, Caroline, 8310 Brugge (BE); De Lathauwer, Geert, 9000 Gent (BE)
(74) Representative: Powis de Tenbossche, Roland

(56) References cited:
- WO-A1-00/61086
- WO-A2-2011/110291
- US-A- 2 890 987
- US-A- 5 773 397
- US-A1- 2001 044 405
- US-B1- 6 765 024
- "Pearlescent concentrate for use in shampoos - obtd. by preparing fatty acid glycol ester soln. by heating with fatty acid alkylolamide as thickener, cooling and crystallising", WPI / THOMSON,, vol. 1983, no. 21, 7 March 1983 (1983-03-07), XP002687977,

## Description

The invention relates to a monoalkanolamide blend with a solid weight content from 30 to 55% by weight while being pourable at temperature ranging at least from 0°C and 30°C.

Enhancing the viscosity of a surfactant formulation has both a marketing and a technical reason. More viscous formulations are considered by the user as often more appropriate and more efficient for cleansing and also allow alternative types of packaging. From technical point of view thickening of a surfactant formulation is advantageous for the application of the formulation on the surface to be cleansed. Furthermore, thickened formulations have the advantage of keeping heterogeneous solutions or suspensions in a stable equilibrium or in condition for achieving a stable equilibrium after a stirring or shaking operation for a short time.

Various mechanisms can be held responsible for a thickening-effect in a surfactant formulation, for example :
- Hydrogen bonding:
- Polymer swelling:
- Chain entangling: polymeric substances like natural gums and modified cellulosic
- Micelle interaction / intermolecular association
- inorganic salts, such as electrolyte salts.

Due to its viscosity-building effect and its foam stabilisation effect, a widely used compound for controlling viscosity of surfactant composition is coconut diethanolamide (Coconut DEA), a condensation product of coconut oil and diethanolamine, said coconut diethanolamide being used or not in combination with an inorganic salt.

More and more objections are raised by formulators as toxicologists pointed out there could be a possible link between Cocamide DEA and carcinogenic activity, possibly due to the presence of some impurities, like N-nitrosodiethanolamine.

This urged manufacturers of raw materials to search towards alternatives to diethanolamide-based surfactants. By now a range of products that mimic the effect of diethanolamides are commercialised:
- PEG-fatty acid esters
- PPG-hydroxyethyl fatty acid amides and its ethoxylated derivatives,
- fatty acid monoalkanolamide ethoxylates
- fatty acid primary alkanolamides with branched alkanolgroups
- a combination of an alkanolamide with short-chain and long-chain alcohols in a surfactant matrix

A main disadvantage of these Cocoamide DEA-replacers is their high production cost and lack of compatibility with formulations when a 1 to 1 replacement is required.

Another solution for avoiding the drawbacks of cocoamide DEA is its replacement by a monoalkanolamide such as for example monoethanolamide or monoisopropanolamide. The practical use or workability of these compounds is however not satisfying since these are marketed as solid flakes, whereby requiring the preparation of the surfactant composition at high temperature.

In order to try to avoid said problem, research has been made for preparing fatty acid monoalkanolamide blends, which can thereafter be cold processed by formulators.
(see for example US 6,765,024)

However formulators have noticed that the composition of such blends is restricted to limited amounts of monoalkanolamides, so as to remain liquid at room temperature.

Tests have shown that when having a too high dosage levels of the monoalkanolamide in the blend, said blend had the state of a quite viscous paste that can no more be processed as a multi-purpose thickener.

It has now been discovered that specific blends while containing high amounts of monoalkanolamide(s) remain liquid and homogeneous, while having a comparable performance in thickening, foaming ability and conditioning as Cocamide DEA.

The monoalkanolamide blend of the invention is a homogeneous monoalkanolamide blend with a solid weight content from 30 to 55% by weight while being pourable at temperature ranging at least from 0°C and 30°C, said blend having a viscosity of less than 10000cps (10000mPa.s), advantageously less than 5000cps (3000mPa.s) at a temperature of 20°C, advantageously for temperature comprised between 10°C and 20°C, most preferably for temperature range comprised between 5°C and 20°C,
said blend comprising :
- 10-40% by weight monoalkanolamide of formula (I)

   R1 - NH - CO - R2

   in which R1 is - (CH₂)ₙ -OH, with n an integer equal to 2, 3 or 4
   R2 is a linear saturated or unsaturated hydrocarbon chain with 5 to 23 carbon atoms
- 17 to 50% of a polyol mixture comprising at least glycerol and at least one or more alkyleneglycols with a molecular weight comprised between 62g and 1 000g, whereby the weight ratio glycerol / alkyleneglycols present in the polyol mixture is comprised between 1 : 50 and 50 : 1; advantageously 1:50 and 20:1, preferably 1:20 and 10:1;
- from 0.1 to 1% by weight alkanolamine comprising 2 to 4 carbon atoms;
- up to 30% by weight of a surfactant selected from amphoteric or zwitterionic surfactants, anionic surfactants and non ionic surfactants and mixtures thereof
- optionally from 0.1 % to 30% by weight fatty acid comprising from 6 to 24 carbon atoms or esters thereof;
- possibly water
whereby the polyol mixture content and the optional water content is/are adapted for ensuring a viscosity of less than 10000cps (10000mPa.s), advantageously less than 5000cps (5000mPa.s), preferably less than 3000cps (3000mPa.s) at a temperature of 20°C, advantageously for temperature comprised between 10°C and 20°C, preferably for temperature range from 5°C and 20°C.

Homogeneous blend means a blend which remain homogeneous and without phase separation for at least one week at 20°C without stirring, advantageously for at least one month, preferably for at least 6 months. The blends of the invention are blends which with gentle stirring or mixing do not have any problems of phase separation.

In the blend of the invention, the polyol mixture when used in appropriate content seems to act as a liquefier for the monoalkanolamide compound present in the blend, advantageously an aqueous blend.

It has been observed that the blend of the invention had excellent performance for viscosity build-up and excellent foaming ability, the blends of the invention having moreover the benefit that these are fluid at room temperature and can be used for cold-processing of cleanser formulations. This means a considerable saving in time and energy.

Preferably, the monoalkanolamide blend comprises from 17% to 70% by weight of aqueous polyol mixture consisting of (a) water and (b) polyol mixture comprising at least glycerol and at least one or more alkyleneglycols with a molecular weight comprised between 62g and 1000g, whereby the weight ratio glycerol / alkyleneglycols present in the polyol mixture is comprised between 1 : 10 and 10 : 1.

When the monoalkanolamide containing mixture contains water, the pH of the mixture at 20°C is advantageously comprised between 7.5 and 10.5, preferably 8 and 10. The pH is for example controlled during the preparation of the blend by adding one or more bases.

According to a preferred embodiment, the monoalkanolamide blend comprises 30% to 60% by weight water and from 5% by weight to 30% by weight of a surfactant selected from amphoteric or zwitterionic surfactants, anionic surfactants and mixtures thereof.

According to advantageous monoalkanolamide blends of the invention, the blends have a solid weight content from 35% to 40% by weight, while having a viscosity of less than 10000 mPa.s (10Pa.s or 10000 cps ), advantageously less than 5000mPa.s (5000 cps), most preferably less than 3000mPa.s (3000cps), at a temperature of 20°C, especially for temperature ranging from 5°C up to 20°C.

According to specific preferred blends of the invention, the blends comprise :
- from 17% to 35% by weight, advantageously from 20% to 30% by weight of a polyol mixture comprising at least glycerol and at least one or more alkyleneglycols with a molecular.weight comprised between 62g and 1000g, whereby the weight ratio glycerol / alkyleneglycols present in the polyol mixture is comprised between 1 : 10 and 10 : 1.

According to details of advantageous embodiments,
- the weight ratio monoalkanolamide of formula (I) / polyol mixture is comprised between 0.5 and 2, and/or
- at least 90% by weight, advantageously at least 95% by weight, preferably at least 97% by weight of the blend consist of monoalkanolamide of formula (I), polyol mixture, surfactant, water and alkanolamine.

The invention relates also to a cold mixing process for preparing an aqueous cleanser containing monoalkanolamide, the process comprising the step of cold mixing a monoalkanolamide blend according to the invention into a separately prepared aqueous formulation comprising at least one surfactant or the step of cold mixing additional compound to a monoalkanolamide blend according to the invention for the preparation of the said aqueous cleanser, whereby the said monoalkanolamide blend formulation provides at least thickening behaviour for the aqueous cleanser.

The invention further relates to a mixing process for preparing a blend according to the invention, the process comprising at least the step of :
- heating (a) a polyol mixture comprising glycerol and at least one or more alkyleneglycols with a molecular weight comprised between 62g and 1000g up to a temperature comprised between 40°C and 90°C, advantageously between 70°C and 80°C,
- optionally adding one or more surfactants, water and/or one or more fatty acids ;
- adding to the polyol mixture having a temperature comprised between 40°C and 90°C, the monoalkanolamide in solid form,
- stirring the mixture for a sufficient time, while maintaining the temperature of said mixture at a level between 40°C and 90°C for melting substantially all monoalkanolamide present into the mixture,
- optionally adding one or more components,
- cooling the monoalkanolamide containing mixture to a temperature below 40°C, while stirring,
- optionally adding one or more components, and
- cooling the monoalkanolamide containing mixture to a temperature below 30°C, while stirring.

Said process further comprises advantageously at least a filtering step after the melting step of substantially all the monoalkanolamide present in the polyol containing mixture, whereby the monoalkanolamide containing mixture is preferably filtered while having a temperature comprised between 40°C and 90°C.

Monoalkanolamides suitable for the blends of the invention include but are not limited to, for example condensation products of monoalkanolamine (monoethanolamine, mono(methylethanol)amine, monopropanolamine, mono(methylpropanol)amine or mixtures thereof)
condensed with one or more fatty acids having from 6 to 24 carbon atoms such as for example caproic acid, caprylic acid, 2-ethylcaproic acid, capric acid, lauric acid, isotridecanoic acid, myristic acid, myristoleic acid, palmitic acid, palmitoleic acid, stearic acid, isostearic acid, oleic acid, elaidic acid, petroselenic acid, linoleic acid, linolenic acid, eleostearic acid, arachidic acid, gadoleic acid, behenic acid and erucic acid or mixtures thereof, and/or with one or more derivatives of such fatty acid(s), such as one or more esters of said fatty acids. The fatty acid(s) or ester(s) thereof is advantageously a saturated fatty acid or a mix of saturated fatty acids or ester(s) thereof. When using a mix of fatty acids or esters, the unsaturated fatty acid content or ester of unsaturated fatty acid content in said mix is advantageously lower than 15% by weight, preferably less than 10% by weight. Most preferred monoalkanolamides are : condensation products of monoethanolamine and/or monoisopropanolamine with one or more fatty acids having 8 to 18 carbon atoms, advantageously with a mix of saturated fatty acids having 8 to 16 carbon atoms, especially 12 and/or 14 carbon atoms, or with one or more esters of said fatty acid(s).

Amphoteric or zwitterionic surfactants suitable for blends of the invention include but are not limited to, for example alkylbetaines, alkylamidobetaines, aminopropionates, aminoglycinates, imidazoliniumbetaines and sulfobetaines. These surfactants are preferred for aqueous blends of the invention, especially for blends containing more than 20% by weight water.

Anionic surfactants suitable for blends of the invention include but are not limited to, for example soaps, alkylbenzenesulfonates, alkanesulfonates, olefmsulfonates, alkylethersulfonates, glycerine ethersulfonates, α-methylester sulfonates, sulfofatty acids, alkylsulfates, fatty alcohol ethersulfates, glycerine ethersulfate, fatty acid ethersulfates, monoglyceride (ether) sulfates, fatty amide (ether) sulfates, mono- and dialkylsulfosuccinates, mono- and dialkylsulfosuccinamates, sulfotriglycerides, ethercarboxylic acids and salts, fatty acid isethionates, fatty acid sarcosinates, fatty acid taurates, N-acylamino acids (acyllactates, acyltartrates, acylglutamates and acylaspartates), alkylglucoside sulphates, protein fatty acid condensates and alkyl (ether) phosphates.

Suitable nonionic surfactants include but are not limited to, for example fatty alcohol polyglycolethers, alkylphenol polyglycolethers, fatty acid polyglycolesters, fatty acid amide polyglycolethers, fatty amine polyglycolethers, alkoxylated triglycerides, alk(ene)ylglycosides, proteinhydrolysates, polyol fatty acid esters, carbohydrate esters, sorbitan ester, polysorbates and aminoxides.

Suitable fatty acids include but are not limited to, fatty acids having from 6 to 24 carbon atoms such as for example caproic acid, caprylic acid, 2-ethylcaproic acid, capric acid, lauric acid, isotridecanoic acid, myristic acid, myristoleic acid, palmitic acid, palmitoleic acid, stearic acid, isostearic acid, oleic acid, elaidic acid, petroselenic acid, linoleic acid, linolenic acid, eleostearic acid, arachidic acid, gadoleic acid, behenic acid and erucic acid or mixtures thereof.

Suitable polyols include but are not limited to, for example glycerine; alkylene glycols such as for example ethylene glycol, diethylene glycol, propylene glycol, butylene glycol, hexylene glycol or polyethylene glycols with an average molecular weight of from 100 to 1.000 Dalton; polyglycerines with a condensation grade of 1.5-10; methylol compounds such as for example trimethylolethane, trimethylolpropane, trimethylolbutane, pentaerythritol and dipentaerythritol; alkylglucosides with 1 to 24 carbon atoms such as for example methyl- and butylglucoside; carbohydrate alcohols with 4 to 12 carbon atoms such as for example sorbitol or mannitol; carbohydrates with 4 to 12 carbon atoms such as for example glucose or saccharose; aminosugars such as for example glucamine; Optionally, but advantageously one or more additives with a liquifying effect on the blend can be used if required, for example for adjusting the viscosity. Among these, typical examples include, but are not limited to, hydroxycarboxylic acids and salts thereof, carboxyamino acids and salts thereof, carboxymethylated derivatives of carboxyamino acids and the corresponding salts thereof, phosphoric acid esters of fatty alcohols or ethoxylated fatty alcohols, quaternary ammonium salts, inorganic salts.

A description of the preparation of blends according to the invention is given hereafter :

### First Example of preparation

- heating (a) a polyol mixture comprising glycerol and at least one or more alkyleneglycols with a molecular weight comprised between 62g and 1000g up to a temperature between 70°C and 80°C. During said heating phase, the polyol mixture is substantially continuously stirred. This operation is for example carried out in a vessel with wall(s) provided with means adapted for heating.
- while maintaining the temperature of the mixture between 70 - 80°C and while stirring the mixture, anionic surfactant(s), amphoteric surfactant(s) and water are introduced into the mixture.
- while maintaining the temperature of the mixture between 70 - 80°C and while stirring the mixture, adding to the mixture the monoalkanolamide (in solid form, for example in the form of flakes),
- stirring the mixture for a sufficient time, while maintaining the temperature of said mixture at a level of 70°C - 80°C so as to have substantially all solid monoalkanolamide molten.
- optional filtering step for removing monoalkanolamide possibly still in solid form or possible aggregates particles present in the liquid mixture.
- optionally adding one or more further components,
- cooling the monoalkanolamide containing mixture to a temperature below 40°C, for example to room temperature, while stirring,
- optional filtering step
- optionally adding one or more further components.

The viscosity of the blend is controlled, and is necessary adjusted by adding further polyol mixture and/or alkyleneglycol(s), followed by a mixing step at a temperature comprised between 40°C and 90°C, for example 70°C - 80°C.

### Second example of preparation

- heating (a) a polyol mixture comprising glycerol and at least one or more alkyleneglycols with a molecular weight comprised between 62g and 1000g up to a temperature between 40°C and 90°C, for example between 40 and 60°C. During said heating phase, the polyol mixture is substantially continuously stirred. This operation is for example carried out in a vessel with wall(s) provided with means adapted for heating.
- while maintaining the temperature of the mixture between 40 - 90°C, for example between 40°C and 60°C, and while stirring the mixture, adding to the mixture the monoalkanolamide (in solid form, for example in the form of flakes),
- stirring the mixture for a sufficient time, while maintaining the temperature of said mixture at a level of 40°C - 90°C so as to have substantially all solid monoalkanolamide molten.
- optional filtering step for removing monoalkanolamide possibly still in solid form or possible aggregates particles present in the liquid mixture.
- while maintaining the temperature of the mixture between 40 - 60°C and while stirring the mixture, adding a nonionic surfactant and a fatty acid. The temperature and stirring are maintained until all components are dissolved.
- optional filtering step
- optionally adding one or more further components, such as further fatty acid(s) and/or colour stabiliser
- cooling the monoalkanolamide containing mixture to a temperature below 40°C, for example to room temperature, while stirring.
- optional adding of water.

The viscosity of the blend is controlled, and is necessary adjusted by adding further polyol mixture and/or alkyleneglycol(s), followed by a mixing step at a temperature comprised between 40°C and 90°C, for example 70°C - 80°C.

Several properties of the blends prepared according to said first or second process are determined, such as viscosity, stability, clearness, thickening behaviour for example in personal care cleansing formulations as well as in household and industrial detergent formulations.

### Example of preparation of monoalkanolamide

While commercial monoalkanolamide can be used for the preparation of blends according to the invention, the monoalkanolamide is advantageously prepared as follows :
Vegetable oil is brought in a vessel and heated at a temperature comprised between 40°C and 75°C. The vessel is provided with a cover so as to carry the heating and mixing steps under inert atmosphere, such as a nitrogen atmosphere. While stirring the hot vegetable oil, monoalkanolamine is added. The mixture while being stirred is maintained at a temperature comprised 40°C and 75°C. An alcali catalyst,
advantageously in the form of an alcoholic solution is added. The reaction mixture is thereafter heated to 95°C under inert atmosphere. The reaction is further processed at these conditions upon a free amine content of less than 3%,
advantageously less than 2%, preferably less than 1% by weight. The amine content can be determined for example by titration. The excess catalyst still present in the mixture is advantageously neutralised with water and/or with one or more mineral or organic acids, advantageously with a solution of one or more acids in water.

The mixture is then cooled to 50°C or less.

Optionally, but advantageously one or more polyols, advantageously one or more alkylene glycols with a molecular weight comprised between 62g and 1000g is/are added to the mixture, before and/or after and/or during the cooling phase of the monoalkanolamide mixture and/or during the neutralisation step of the excess of catalyst.

### Specific Example of production of monoalkanolamide

A vegetable oil (1268 kg) is brought in a reaction vessel, covered under gaseous nitrogen blanket and stirred for about 30 minutes. Monoalkanolamine (414 kg) is added to the reaction vessel. The mixture is stirred, heated upto 40°C and an alkali catalyst (e.g. 30% methanolic sodium methylate solution) (20 kg) is added. The reaction mixture is further stirred and heated comprised between 90°C - 95°C. The reaction is further processed at these conditions upon a free amine content of less than 2%. The amine content can be determined for example by titration. The excess catalyst still present in the mixture is advantageously neutralised with water (4 kg) and one or more mineral or organic acids. Then the product is cooled to 50°C and eventually polyol is added (2000 kg) resulting in a clear yellow oil which is used in the fluid state for consecutive production of the blend of the invention.

Coconut oil and/or palm oil and/or copra oil and mixtures thereof are examples of oils suitable for the preparation of the monoalkanolamide. Said oil(s) is /are advantageously hydrogenated and/or refined and/or hydrogenated. Preferably the oil used is rich in saturated lauric and myristic fatty acids, advantageously the oil used comprises more than 50% by weight, preferably more than 65% by weight of lauric and myristic fatty acids.

The monoalkanolamine used is for example monoethanolamine, monopropanolamine, mono isopropanolamine and mixtures thereof.

As used in the tables, the following abbreviations have the following meaning:
- coco MEA : Cocoamide MEA or coco monoethanolamide being the condensation product of refined and/or (preferably and) hydrogenated coconut oil with mono ethanol amine;
- Coco MIPA : Cocoamide MIPA or coco mono isopropanol amide being the condensation product of refined and/or (preferably and) hydrogenated coconut oil with isopropanol amine;
- C12 to C14 coco MEA : Cocoamide MEA or coco monoethanolamide being the condensation product of the fraction with 12 and 14 carbon atoms of refined and hydrogenated coconut oil with mono ethanol amine;
- C12 to C14 Coco MIPA : Cocoamide MIPA or coco mono isopropanol amide being the condensation product of refined and hydrogenated coconut oil with isopropanol amine;

### Specific Examples of monoalkanolamide blends

### Example 1

A polyol (162 kg) is brought into the reaction vessel and heated up to a temperature of 70-75°C under continuous stirring, while also an anionic surfactant (260 kg) and an amphoteric surfactant (65 kg) in water (32 kg) are introduced into the mixture.

After said mixing being carried out and while the temperature of the mixture is still in the range of 70 - 75°C, solid monoalkanolamide (130 kg) is progressively added. The blend is mixed for at least 2 hours at this temperature in order to allow all solid components to be molten. The blend was then cooled to room temperature under continuous stirring until a temperature of 25°C is reached. The blend, optionally after addition of one or more additives, had a pH of about 8-10. The viscosity of the blend was lower than 5000 cps (measured according to Brookfield RVF 3/4).

### Example 2

A polyol (613 kg) is brought into a reaction vessel and heated up to 40°C. A mixture of a solid monoalkanolamide and polyol (3717 kg) is added. This mixture is stirred and heated up to 40°C. After melting of the monoalkanolamide, a nonionic surfactant (1493 kg) and a fatty acid (299 kg) are added. The temperature is kept at 40°C until all components are dissolved. At a temperature between 35 and 40 °C extra fatty acid (1344 kg) is added. The clearness is checked and the mixture is cooled to room temperature resulting in a clear yellowish fluid. The pH of the blend (after dilution in organic solvent, the blend corresponding to 5% by weight) was controlled (for example by adding one or more additives) so as to be in the range of 5.0 to 7.0.

The viscosity of the blend was lower than 1000cps, advantageously lower than 250 cps (measured according to Brookfield RVF 3/4).

### Examples of blends

Various blends of the invention, as well as comparative blends have been prepared, the composition of which (% by weight, as dry matter or active content present in the blend) is given in the following tables.

In said compositions, the following ingredients have been used :
- monoalkanolamide : coco MEA, Coco MIPA, C12 to C14 coco MEA, C12 to C 14 Coco MIPA and mixtures thereof
- polyol : propylene glycol, diethylene glycol, butylene glycol, hexylene glycol and mixtures thereof
- alcohol (mono) : ethanol, propanol and mixtures thereof
- amphoteric surfactant: cocoamido propyl betaine (cocamidopropyl betaine), sodium cocoamphoacetate and mixtures thereof
- anionic surfactant: potassium cocoate, sodium laureth sulfate, sodium myristoyl sarcosinate, and mixtures thereof
- non ionic surfactant: PPG-1-PEG-9-lauryl glycol ether ( POLYOXYPROPYLEN-1- POLYOXYETHYLEN-9- DODECYL GLYCOL ETHER ), sorbitan laurate, and mixtures thereof
- fatty acid : saturated fatty acids with 12 to 18 carbon atoms (C12-18 fatty acid meaning a mixture of fatty acids with 12 to 18 carbon atoms; while C12 fatty acid means fatty acid with 12 carbon atoms)
- water : demineralized water

### Examples of homogeneous blends according to the invention

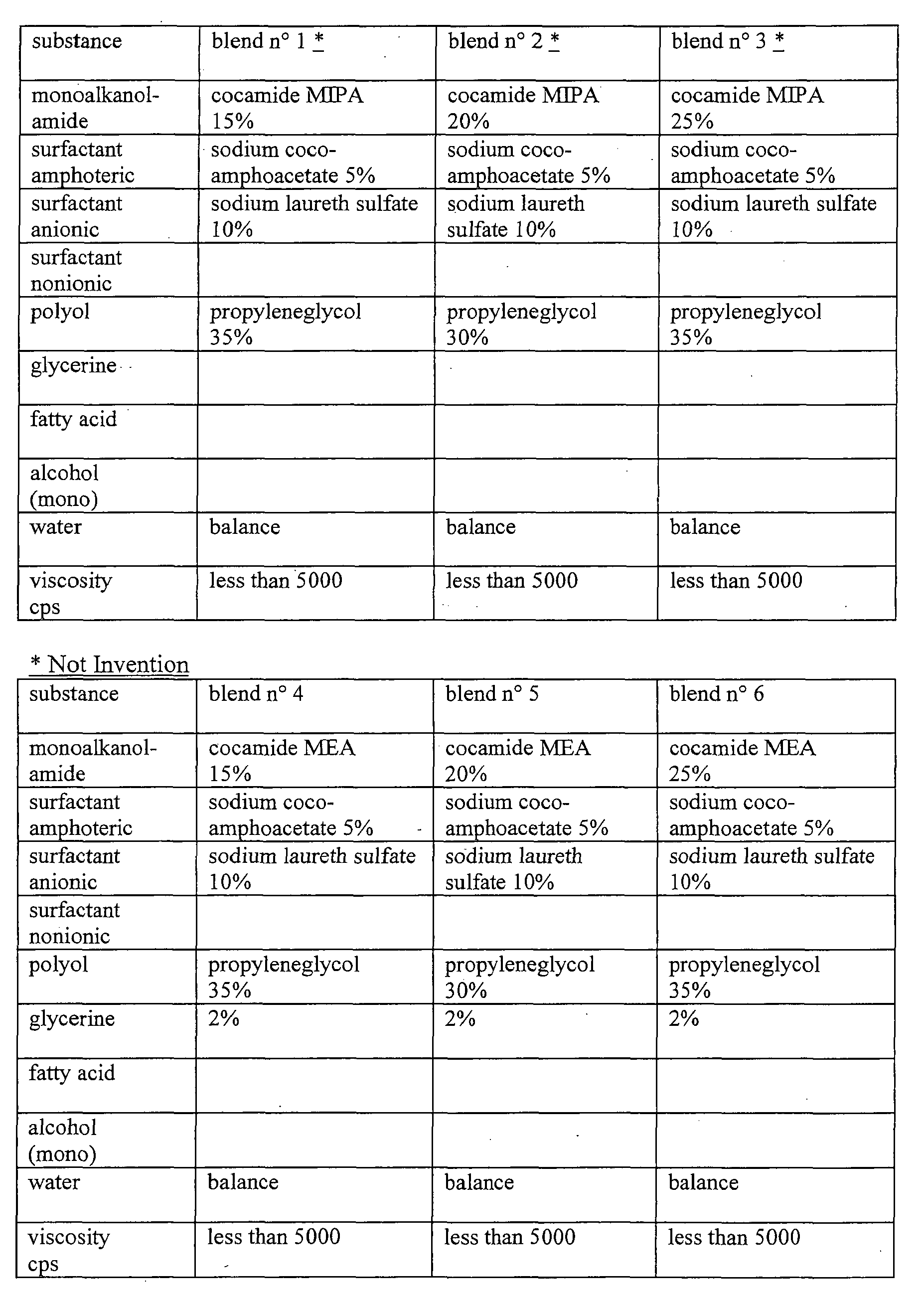

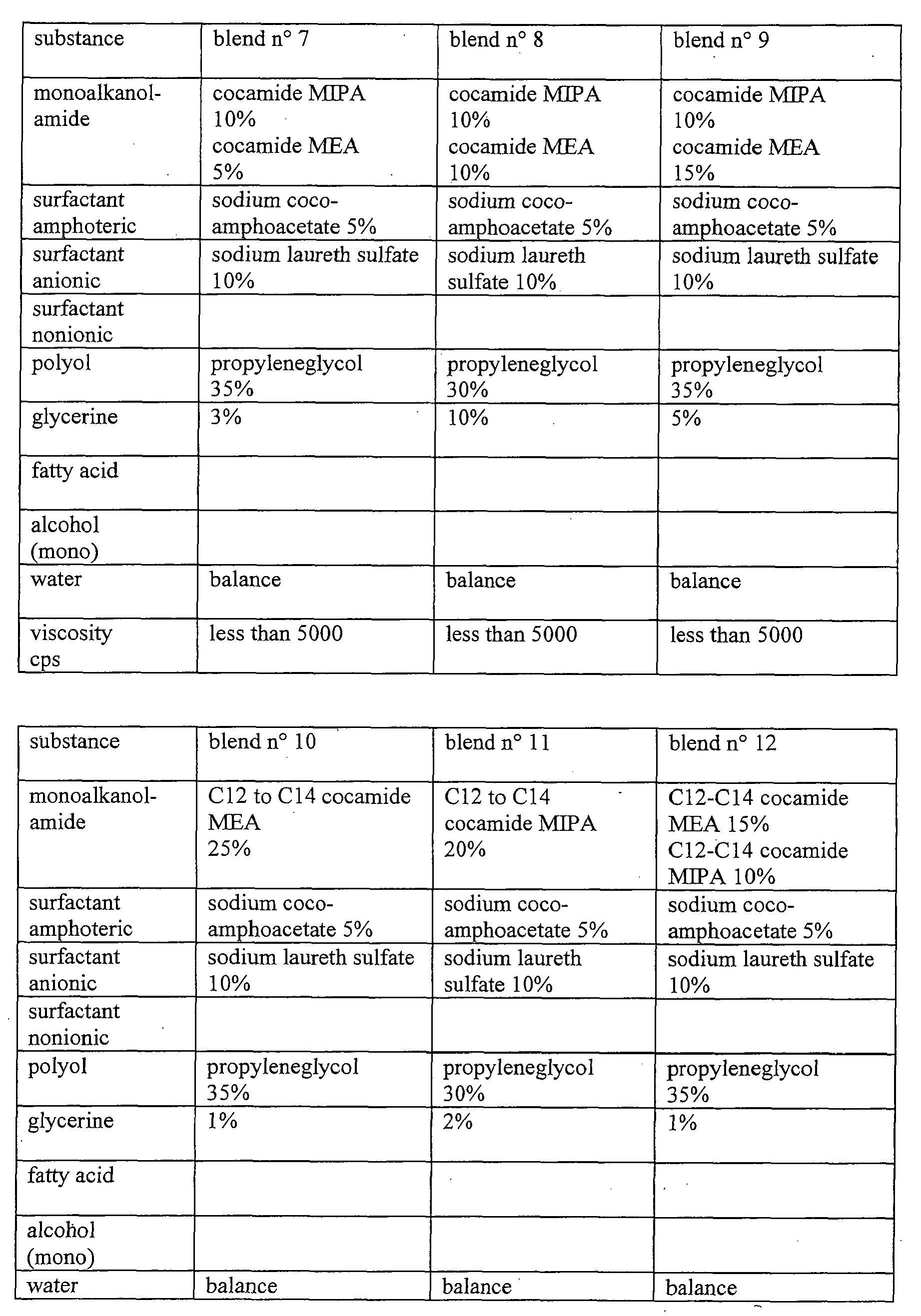

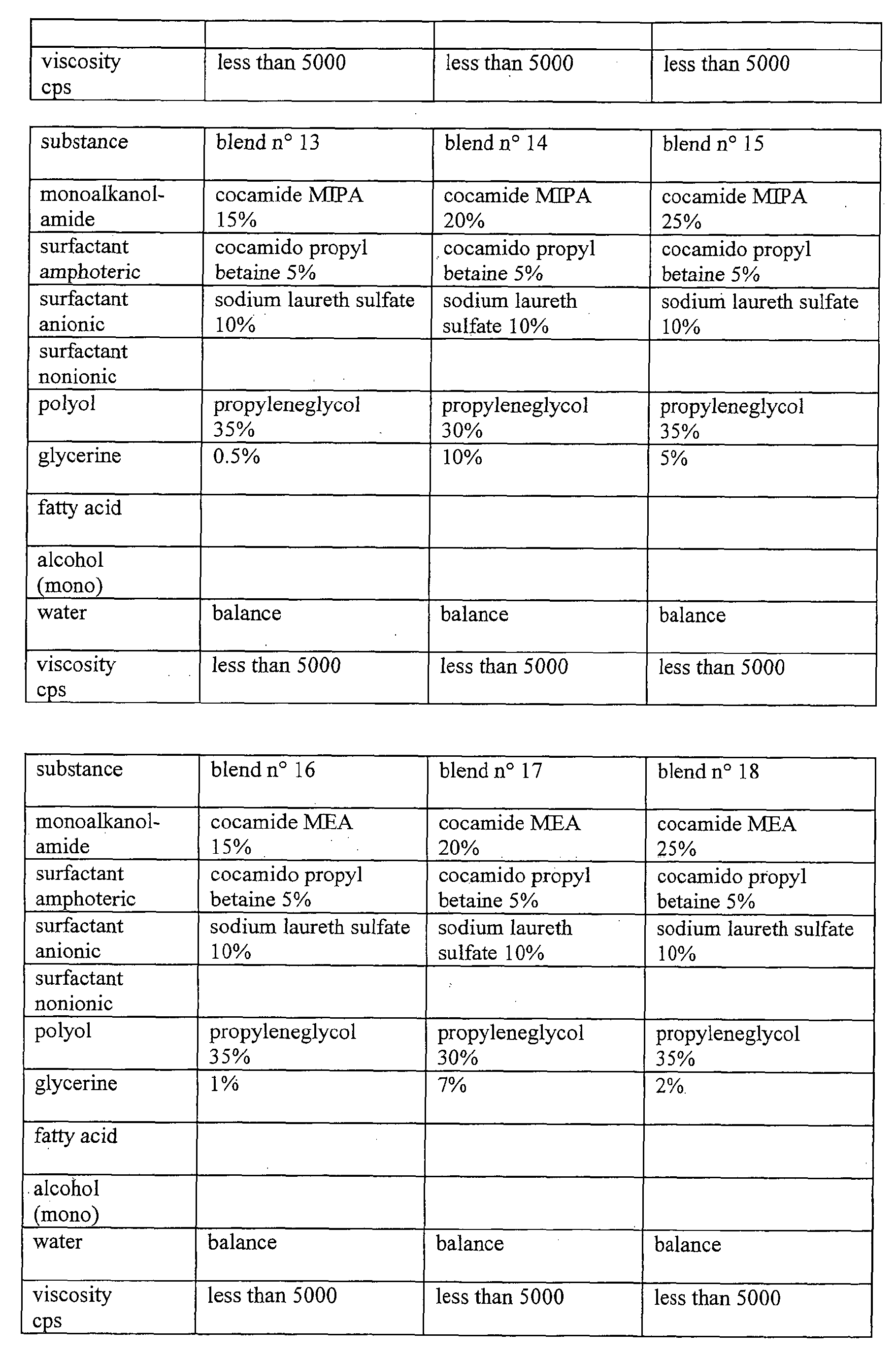

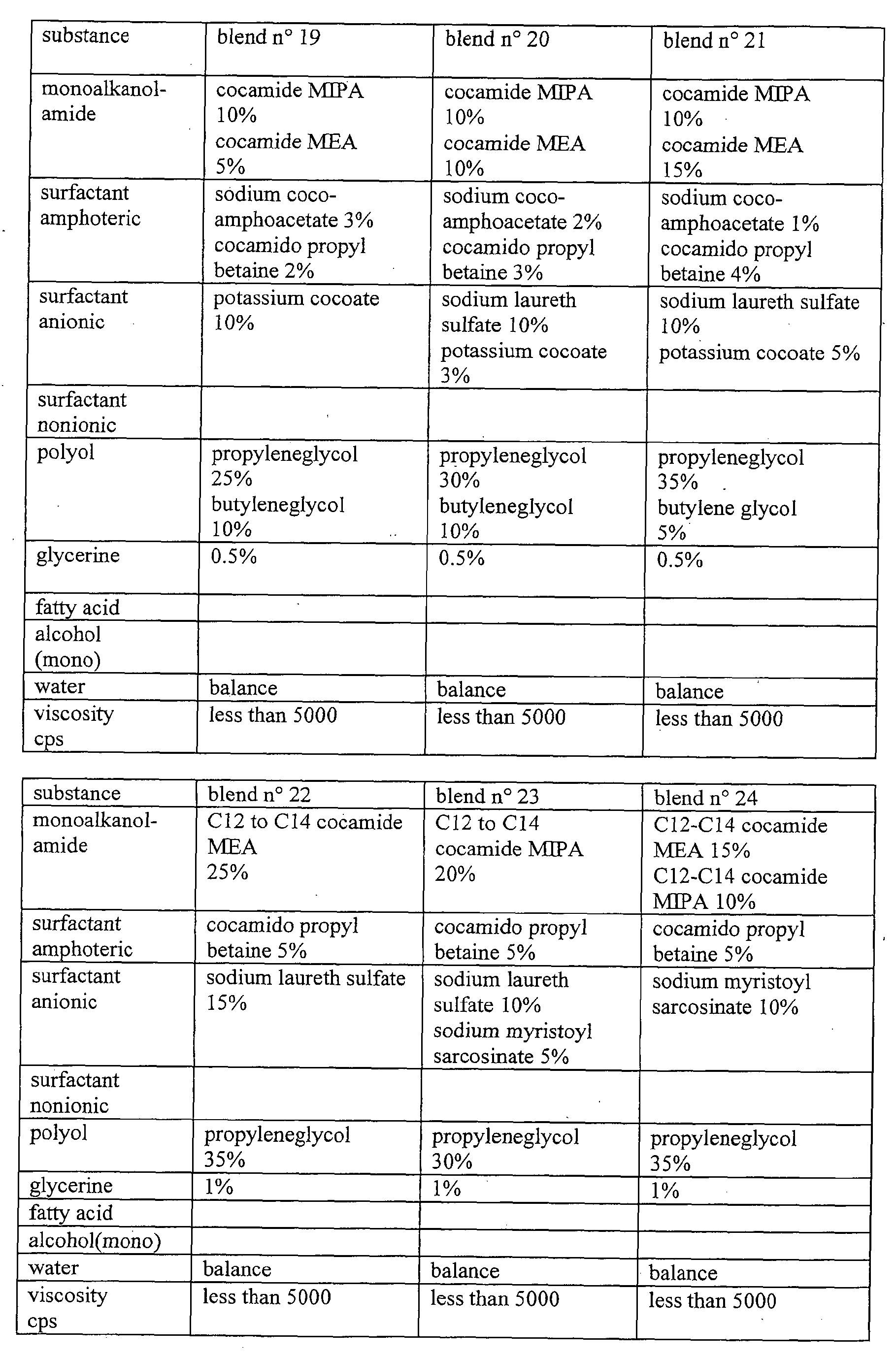

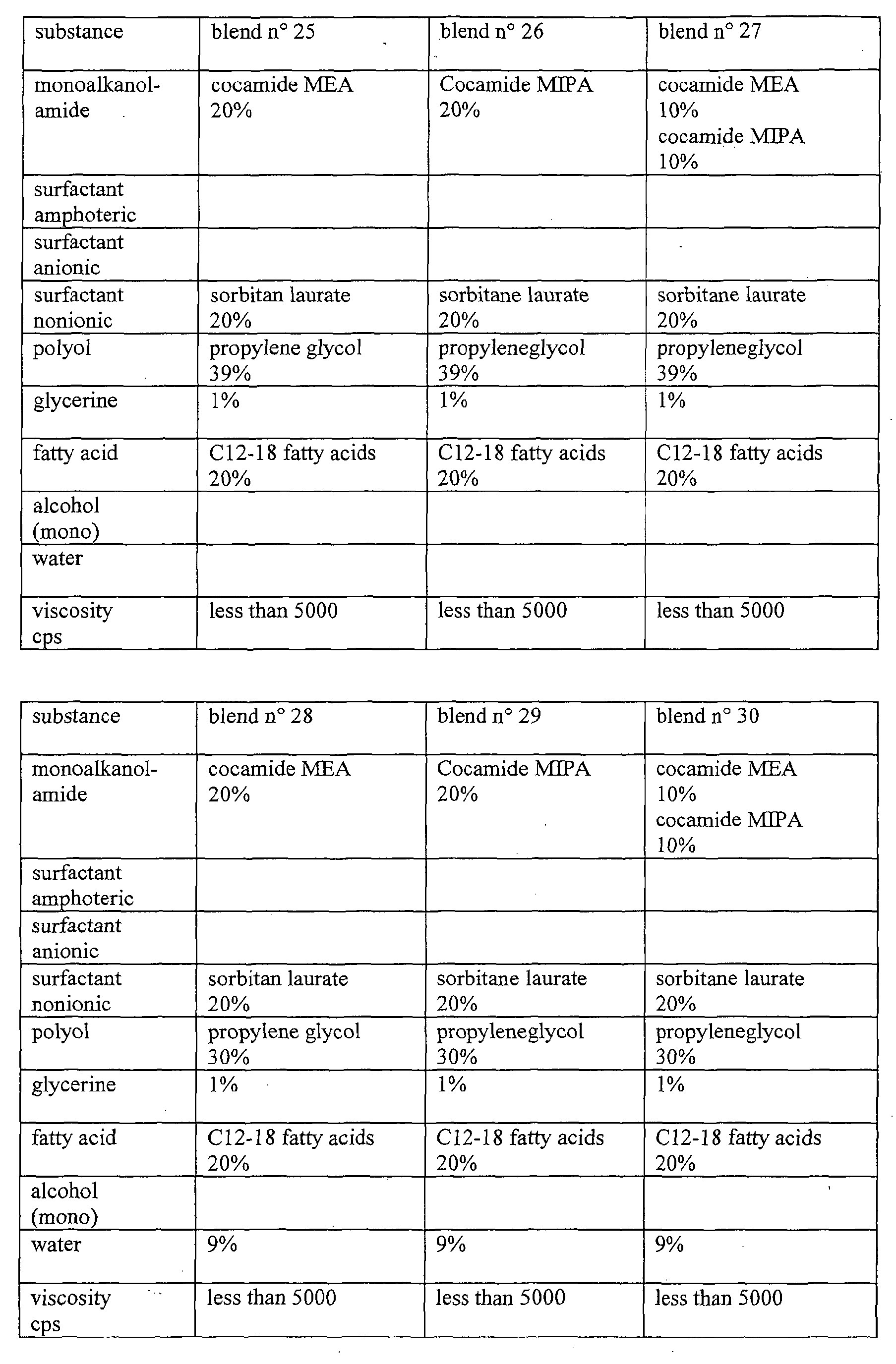

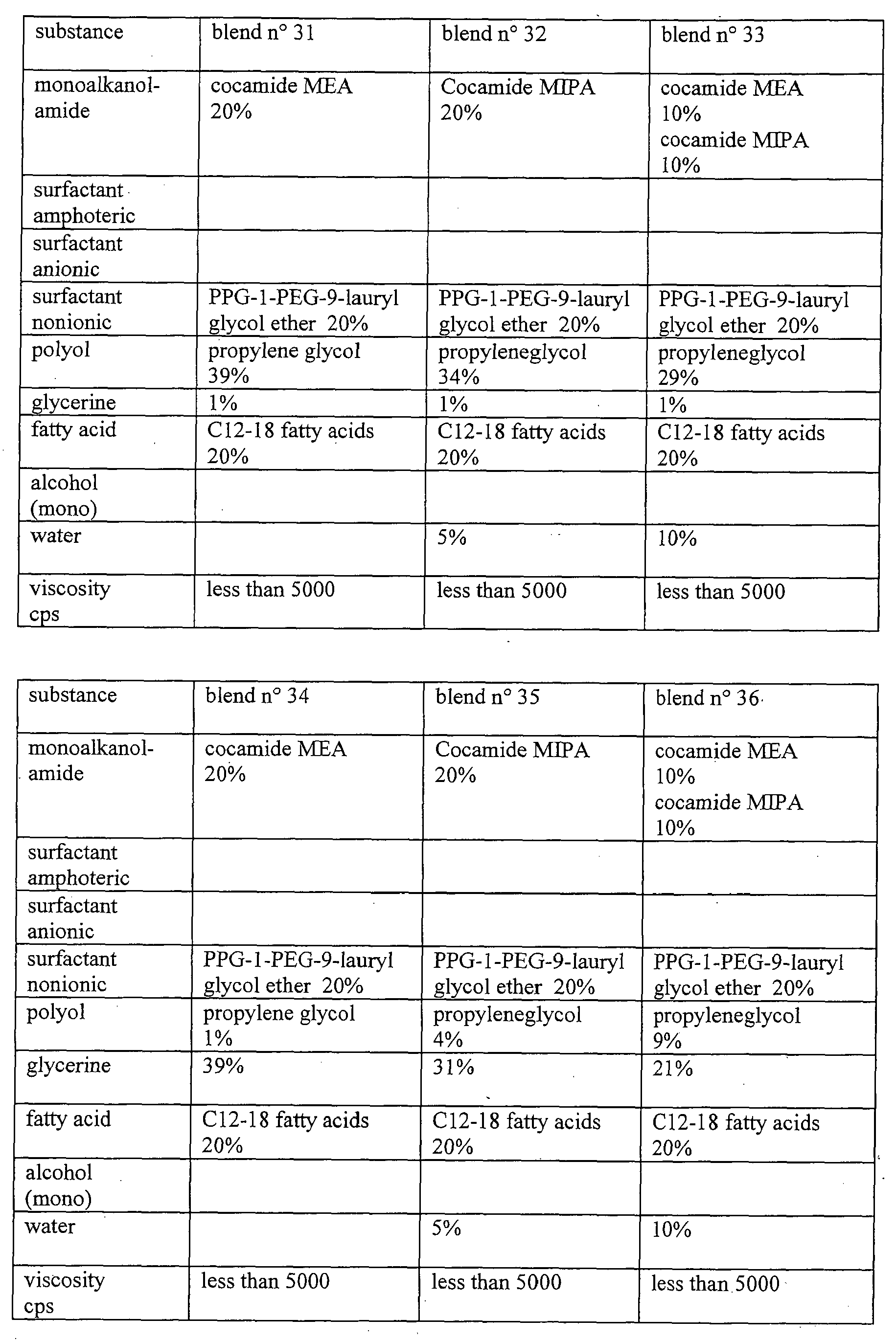

The viscosity of blends according to the invention was measured at 20°C.

The viscosity was further measured at 10°C. The measured viscosity was still below 5000cps, especially below 3000cps, more specifically below about 1500cps.

### Comparative examples (not according to the invention)

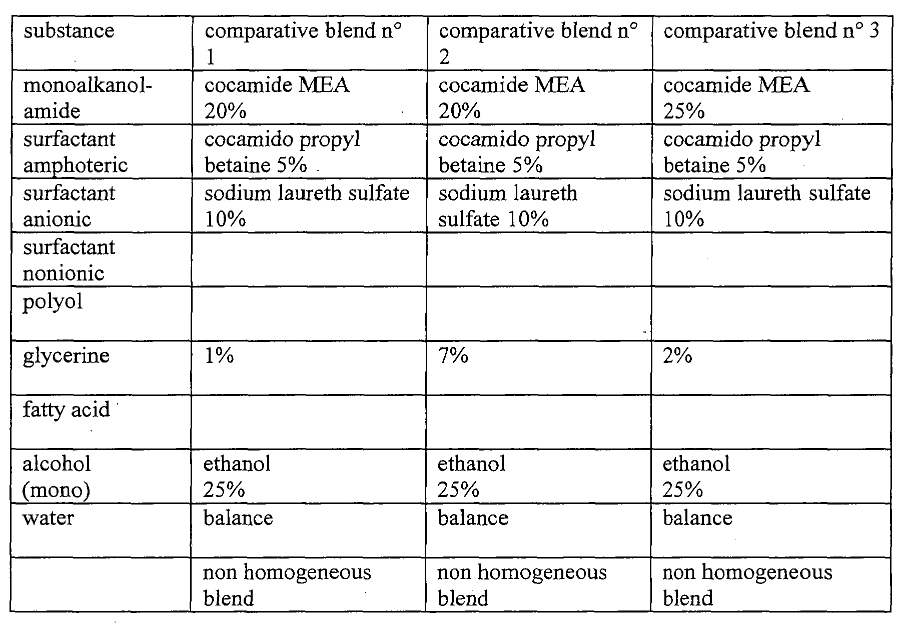

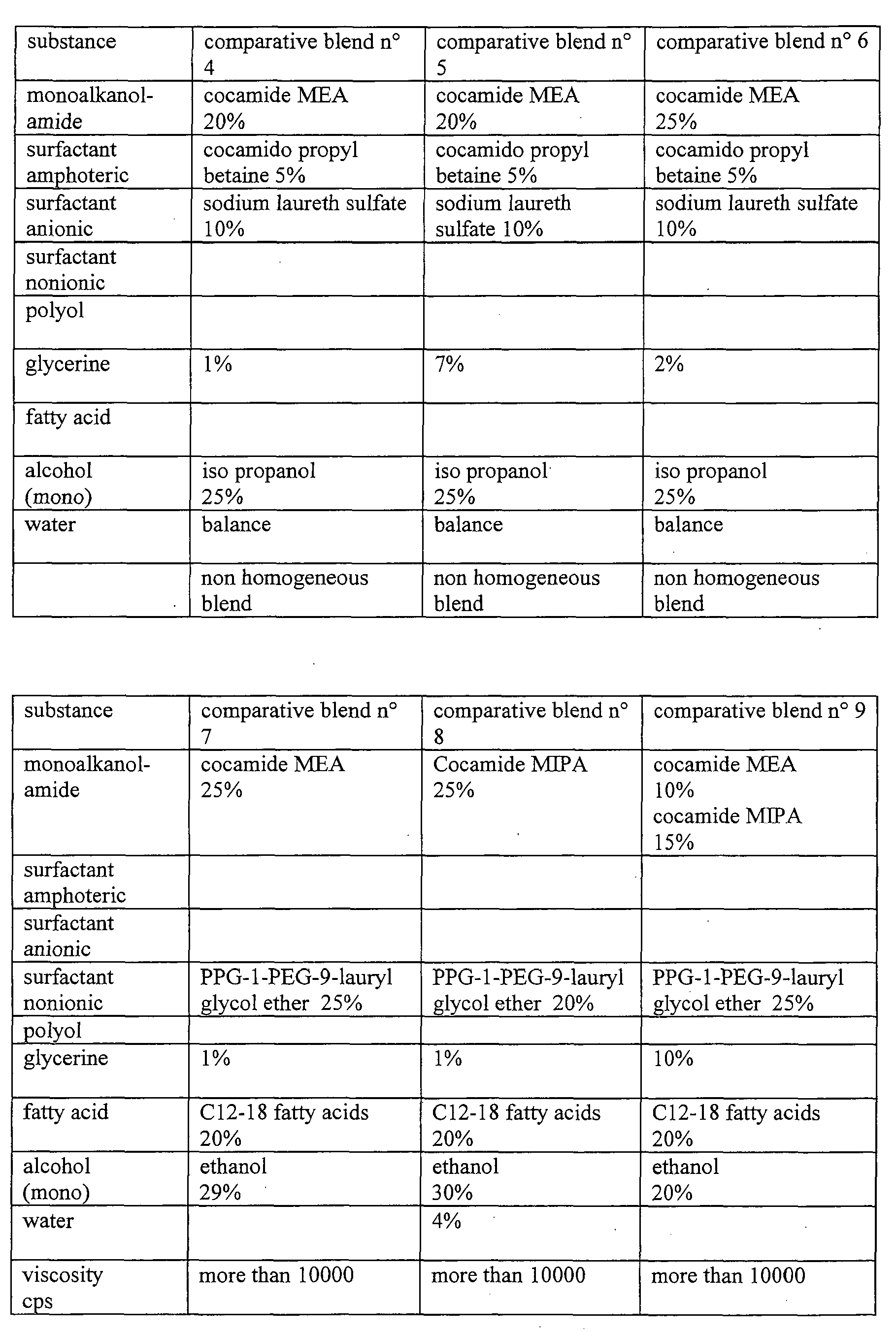

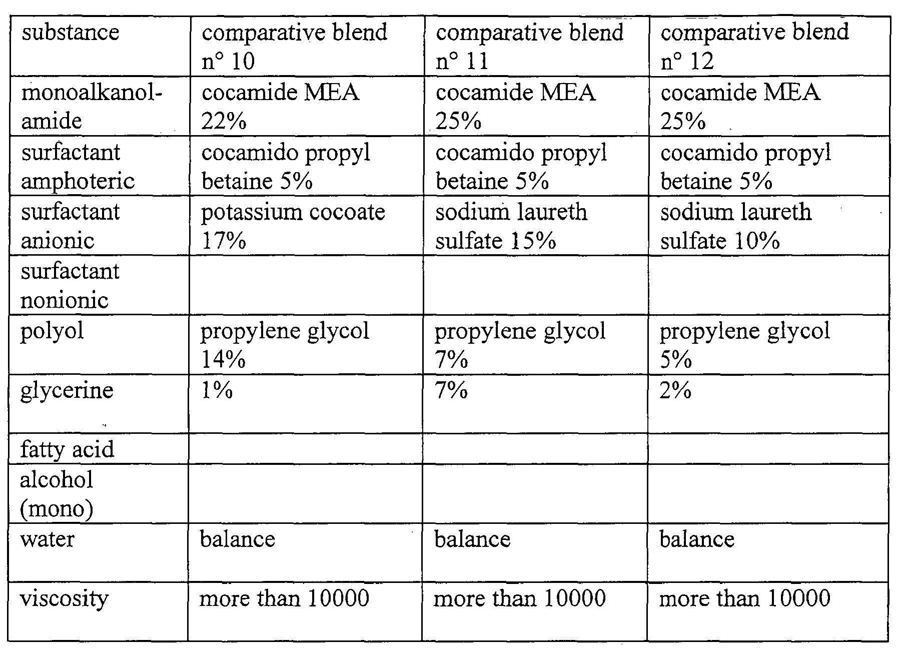

For comparative purpose, alcohol (pure ethanol) was added to the blends n°1 to n°20, so that the ethanol content was 20% by weight. The so obtained blends were not homogeneous.

### Examples formulation of aqueous monoalkanolamide blend

The aqueous monoalkanolamide blend of the invention can be used in typical cleansing formulations, such as shampoo's, liquid dish wash formulations, body cleanser.

For example, the blends of the invention can be used in the preparation of cleansing formulations comprising (expressed as active agent or dry weight for the blend of the invention) : Sodium Laureth Sulfate (8 to 15% by weight), aqueous monoalkanolamide blend of the invention (for example from 5 to 15% by weight), Cocamidopropyl Betaine (1 to 10% by weight), Glycerine (0 to 5% by weight), additives (0 to 10% by weight) and an amount of water upto 100%.

The pH of the mixture is adjusted, for example with citric acid to pH comprised between 4 and 5.5, while the viscosity of the mixture can be adjusted for example by adding Sodium Chloride, to achieve a viscosity 2200cps (2200 mPa.s) (Brookfield RVF 3/4).

### Examples formulation of non-aqueous monoalkanolamide blend

The non-aqueous monoalkanolamide blend of the invention can be used in a typical cleansing formulations, such as shampoo's, liquid dish wash formulations, body cleanser.

For example, the blends of the invention can be used in the preparation of cleansing formulations comprising (expressed as active agent or dry weight for the blend of the invention): Coco-Betaine (for example from 15 to 30% by weight), Disodium Laureth Sulfosuccinate (from 10% to 20% by weight), Sodium Cocoamphoacetate (from 5% to 15% by weight), Glycerine (from 1% to 5% by weight), PEG-7 Glyceryl Cocoate (from 0.5% to 5% by weight), non-aqueous monoalkanolamide blend of the invention (from 0.5% to 5% by weight), additives (from 0 to 15% by weight) and water upto 100%. The pH is advantageously adjusted to 5-6, such as about 5.5, advantageously with lactic acid.

The so prepared formulation can be mixed/dissolved in Isobutane and/or Propane and/or Butane in order to be used as aerosol, for example in a shower mousse.

## Claims

1. Homogeneous monoalkanolamide blend with a solid weight content from 30% to 55% by weight while being pourable at temperature ranging at least from 0°C and 30°C, said blend having a viscosity of less than 10000cps (10000mPa.s), advantageously less than 5000cps (5000mPa.s) at a temperature of 20°C, advantageously for temperature comprised between 10°C and 20°C,
said blend comprising :
- 10-40% by weight monoalkanolamide of formula (I)
R1-NH-CO-R2
in which R1 is - (CH₂)ₙ -OH, with n an integer equal to 2, 3 or 4
R2 is a linear saturated or unsaturated hydrocarbon chain with 5 to 23 carbon atoms
- 17 to 50% of a polyol mixture comprising at least glycerol and at least one or more alkyleneglycols with a molecular weight comprised between 62g and 1000g, whereby the weight ratio glycerol / alkyleneglycols present in the polyol mixture is comprised between 1 : 50 and 50 : 1, advantageously between 1 : 50 and 20 : 1;
- from 0.1 to 1% by weight alkanolamine comprising 2 to 4 carbon atoms;
- up to 30% by weight of a surfactant selected from amphoteric or zwitterionic surfactants, anionic surfactants and non ionic surfactants and mixtures thereof
- optionally from 0.1% to 30% by weight fatty acid comprising from 6 to 24 carbon atoms;
- possibly water
whereby the water content and/or polyol mixture content is adapted for ensuring a viscosity of less than 10000cps (10000mPa.s), advantageously less than 5000cps (5000mPa.s) at a temperature of 20°C, advantageously for temperature comprised between 10°C and 20°C.

2. The monoalkanolamide blend of claim 1, which comprises from 17 to 70% by weight water and polyol mixture comprising at least glycerol and at least one or more alkyleneglycols with a molecular weight comprised between 62g and 1000g, whereby the weight ratio glycerol / alkyleneglycols present in the polyol mixture is comprised between 1 : 50 and 50 : 1, advantageously between 1:50 and 20:1.

3. The monoalkanolamide blend of claim 1 or 2, which comprises 30% to 60% by weight water and from 5% by weight to 30% by weight of a surfactant selected from amphoteric or zwitterionic surfactants, anionic surfactants and mixtures thereof.

4. The monoalkanolamide blend of anyone of the preceding claims having a solid weight content from 35% to 40% by weight, while having a viscosity of less than 10000cps (100OOmPa.s), advantageously less than 5000cps (5000mPa.s) at a temperature of 20°C, advantageously for temperature comprised between 10°C and 20°C.

5. The monoalkanolamide blend of anyone of the preceding claims, which comprises :
- from 17% to 35% by weight, advantageously from 20% to 30% by weight of a polyol mixture comprising at least glycerol and at least one or more alkyleneglycols with a molecular weight comprised between 62g and 1000g, whereby the weight ratio glycerol / alkyleneglycols present in the polyol mixture is comprised between 1 : 50 and 50 : 1, advantageously between 1:50 and 20:1.

6. The monoalkanolamide blend of anyone of the preceding claims, in which the weight ratio monoalkanolamide of formula (I) / polyol mixture is comprised between 0.5 and 2.

7. The monoalkanolamide blend of anyone of the preceding claims, in which at least 90% by weight, advantageously at least 95% by weight, preferably at least 97% by weight of the blend consist of monoalkanolamide of formula (I), polyol mixture, surfactant, water and alkanolamine.

8. A cold mixing process for preparing an aqueous liquid cleanser containing monoalkanolamide, the process comprising the step of cold mixing a monoalkanolamide blend according to anyone of the preceding claims into a separately prepared aqueous formulation comprising at least one surfactant or the step of cold mixing additional compound to a monoalkanolamide blend according to anyone of the preceding claims for the preparation of the said aqueous liquid cleanser, whereby the said monoalkanolamide blend formulation provides at least thickening behaviour for the aqueous liquid cleanser.

9. A mixing process for preparing a blend according to anyone of the claims 1 to 7 , the process comprising at least the step of :
- heating (a) a polyol mixture comprising glycerol and at least one or more alkyleneglycols with a molecular weight comprised between 62g and 1000g , up to a temperature comprised between 40°C and 90°C, advantageously between 70°C and 80°C,
- optionally adding one or more surfactants, water and/or one or more fatty acids ;
- adding to the polyol mixture having a temperature comprised between 40°C and 90°C, the monoalkanolamide in solid form,
- stirring the mixture for a sufficient time, while maintaining the temperature of said mixture at a level between 40°C and 90°C for melting substantially all monoalkanolamide present into the mixture,
- optionally adding one or more components,
- cooling the monoalkanolamide containing mixture to a temperature below 40°C, while stirring,
- optionally adding one or more components, and
- cooling the monoalkanolamide containing mixture to a temperature below 30°C, while stirring.

10. The process of the preceding claim, which further comprises at least a filtering step after the melting step of substantially all the monoalkanolamide present in the polyol containing mixture.

11. The process of the preceding claim, in which the monoalkanolamide containing mixture is filtered while having a temperature comprised between 40°C and 90°C.

## Patentansprüche

1. Homogene Monoalkanolamid-Mischung mit einem Feststoffgehalt von 30 Gew.-% bis 55 Gew.-%, welche in einem Temperaturbereich von wenigstens im Bereich von 0 °C und 30 °C gießfähig ist, wobei die Mischung eine Viskosität von weniger als 10000 cps (10000 mPa.s), vorteilhafterweise von weniger als 5000 cps (5000 mPa.s) bei einer Temperatur von 20 °C, vorteilhafterweise für eine Temperatur umfasst zwischen 10 °C und 20 °C aufweist, wobei die Mischung umfasst:
- 10-40 Gew.-% Monoalkanolamid der Formel (I)
R1-NH-CO-R2,
in welcher R1 -(CH₂)ₙ-OH ist, wobei n eine Ganzzahl ist, welche gleich 2, 3 oder 4 ist,
R2 eine lineare gesättigte oder ungesättigte Kohlenwasserstoffkette mit 5 bis 23 Kohlenstoffatomen ist,
- 17 bis 50 % einer Polyolmischung umfassend wenigstens Glycerin und wenigstens ein oder mehrere Alkylenglykole mit einem Molekulargewicht umfasst zwischen 62 g und 1000 g, wobei das in der Polyolmischung vorhandene Gewichtsverhältnis Glycerin/Alkylenglykole zwischen 1:50 und 50:1 umfasst ist, vorteilhafterweise zwischen 1:50 und 20:1;
- von 0,1 bis 1 Gew.-% Alkanolamin umfassend 2 bis 4 Kohlenstoffatome,
- bis zu 30 Gew.-% einer oberflächenaktiven Substanz ausgewählt aus amphoteren oder zwitterionischen oberflächenaktiven Substanzen, anionischen oberflächenaktiven Substanzen und nichtionischen oberflächenaktiven Substanzen und Mischungen davon,
- gegebenenfalls von 0,1 Gew.-% bis 30 Gew.-% Fettsäure umfassend von 6 bis 24 Kohlenstoffatome;
- möglicherweise Wasser,
wobei der Wassergehalt und/oder der Gehalt der Polyolmischung so angepasst ist, dass er eine Viskosität von weniger als 10000 cps (10000 mPa.s) sicherstellt, vorteilhafterweise von weniger als 5000 cps (5000 mPa.s) bei einer Temperatur von 20 °C, vorteilhafterweise für eine Temperatur umfasst zwischen 10 °C und 20 °C.

2. Monoalkanolamid-Mischung nach Anspruch 1, umfassend von 17 bis 70 Gew.-% Wasser und Polyolmischung umfassend wenigstens Glycerin und wenigstens ein oder mehrere Alkylenglykole mit einem Molekulargewicht umfasst zwischen 62 g und 1000 g, wobei das in der Polyolmischung vorhandene Gewichtsverhältnis Glycerin/Alkylenglykole zwischen 1:50 und 50:1 umfasst ist, vorteilhafterweise zwischen 1:50 und 20:1.

3. Monoalkanolamid-Mischung nach Anspruch 1 oder 2, umfassend 30 Gew.-% bis 60 Gew.-% Wasser und 5 Gew.-% bis 30 Gew.-% einer oberflächenaktiven Substanz ausgewählt aus amphoteren oder zwitterionischen oberflächenaktiven Substanzen, anionischen oberflächenaktiven Substanzen und Mischungen davon,

4. Monoalkanolamid-Mischung nach einem der vorhergehenden Ansprüche, welche einen Feststoffgehalt von 35 Gew.-% bis 40 Gew.-% aufweist, wobei sie eine Viskosität von weniger als 10000 cps (10000 mPa.s) aufweist, vorteilhafterweise von weniger als 5000 cps (5000 mPa.s) bei einer Temperatur von 20 °C, vorteilhafterweise für eine Temperatur umfasst zwischen 10 °C und 20 °C.

5. Monoalkanolamid-Mischung nach einem der vorhergehenden Ansprüche, umfassend
- von 17 bis 35 Gew.-% , vörteilhafterweise von 20 Gew.-% bis 30 Gew% einer Polyolmischung umfassend wenigstens Glycerin und wenigstens ein oder mehrere Alkylenglykole mit einem Molekulargewicht umfasst zwischen 62 g und 1000 g, wobei das in der Polyolmischung vorhandene Gewichtsverhältnis Glycerin/Alkylenglykole zwischen 1:50 und 50:1 1 umfasst ist, vorteilhafterweise zwischen 1:50 und 20:1.

6. Monoalkanolamid-Mischung nach einem der vorhergehenden Ansprüche, in welcher das Gewichtsverhältnis Monoalkanolamid der Formel (I) / Polyolmischung zwischen 0,5 und 2 umfasst ist.

7. Monoalkanolamid-Mischung nach einem der vorhergehenden Ansprüche, in welcher wenigstens 90 Gew.-%, vorteilhafterweise wenigstens 95 Gew.-%, vorzugsweise wenigstens 97 Gew.-% der Mischung aus Monoalkanolamid der Formel (I), Polyolmischung, oberflächenaktiver Substanz, Wasser und Alkanolamin bestehen.

8. Kaltmischverfahren zur Herstellung eines wässrigen flüssigen Reinigungsmittels enthaltend Monoalkanolamid, wobei das Verfahren den Schritt eines kalten Mischens einer Monoalkanolamid-Mischung nach einem der vorhergehenden Ansprüche in eine separat hergestellte wässrige Formulierung umfassend wenigstens eine oberflächenaktive Substanz, oder den Schritt eines kalten Mischens einer zusätzlichen Verbindung mit einer Monoalkanolamid-Mischung nach einem der vorhergehenden Ansprüche umfasst, zur Herstellung des wässrigen flüssigen Reinigungsmittels, wobei die Monoalkanolamid-Mischungsformulierung wenigstens ein Verdickungsverhalten für das wässrige flüssige Reinigungsmittel bereitstellt.

9. Mischverfahren zur Herstellung einer Mischung nach einem der Ansprüche 1 bis 7, wobei das Verfahren wenigstens den Schritt umfasst:
- Erwärmen (a) einer Polyolmischung umfassend wenigstens Glycerin und wenigstens ein oder mehrere Alkylenglykole mit einem Molekulargewicht umfasst zwischen 62 g und 1000 g, auf eine Temperatur umfasst zwischen 40 °C und 90 °C, vorteilhafterweise zwischen 70 °C und 80 °C,
- gegebenenfalls Hinzufügen von einer oder mehreren oberflächenaktiven Substanzen, Wasser und/oder einer oder mehreren Fettsäuren;
- Hinzufügen des Monoalkanolamids in fester Form zu der Polyolmischung, welche eine Temperatur umfasst zwischen 40 °C und 90 °C aufweist,
- Rühren der Mischung für eine ausreichende Zeit, wobei die Temperatur der Mischung auf einem Niveau zwischen 40 °C und 90 °C gehalten wird, zum Schmelzen von im Wesentlichen des ganzen Monoalkanolamids, welches in der Mischung vorhanden ist,
- gegebenenfalls Hinzufügen von einem oder mehreren Bestandteilen,
- Kühlen der Monoalkanolamid enthaltenden Mischung auf eine Temperatur unterhalb von 40 °C, wobei gerührt wird,
- Gegebenenfalls Hinzufügen von einem oder mehreren Bestandteilen, und
- Kühlen der Monoalkanolamid enthaltenden Mischung auf eine Temperatur unterhalb von 30 °C, wobei gerührt wird.

10. Verfahren nach dem vorhergehenden Anspruch, welches weiterhin wenigstens einen Filterschritt nach dem Schmelzschritt von im Wesentlichen des ganzen Monoalkanolamids, welches in der Polyol enthaltenden Mischung vorhanden ist, umfasst.

11. Verfahren nach dem vorhergehenden Anspruch, in welchem die Monoalkanolamid enthaltene Mischung gefiltert wird, wobei diese eine Temperatur umfasst zwischen 40 °C und 90 °C aufweist.

## Revendications

1. Mélange homogène de monoalkanolamide ayant une teneur en matières solides comprise entre 30 % et 55 % en poids, tout en étant versable à une température comprise entre au moins 0 °C et 30 °C, ledit mélange ayant une viscosité inférieure à 10 000 cps (10 000 mPa.s), avantageusement inférieure à 5 000 cps (5 000 mPa..s) à une température de 20 ° C, avantageusement à une température comprise entre 10 °C et 20 °C, ledit mélange comprenant :
- de 10 à 40 % en poids du monoalkanolamide de formule (I)
R1 -NH-CO-R2
où R1 est - (CH₂)ₙ -OH, n étant un entier égal à 2, 3 ou 4
R2 est une chaîne hydrocarbure saturée ou insaturée linéaire possédant de 5 à 23 atomes de carbone
- de 17 à 50 % d'un mélange de polyols comprenant au moins un glycérol et au moins un ou plusieurs alkylèneglycols avec une masse moléculaire comprise entre 62 g et 1 000 g, le rapport pondéral entre le glycérol et les alkylèneglycols présents dans le mélange de polyols étant compris entre 1:50 et 50:1, avantageusement entre 1:50 et 20:1 ;
- de 0,1 à 1 % en poids d'alkanolamine comprenant de 2 à 4 atomes de carbone ;
- jusqu'à 30 % en poids d'un tensioactif sélectionné parmi les tensioactifs amphotères ou zwitterioniques, les tensioactifs anioniques et les tensioactifs non ioniques ainsi que les mélanges de ceux-ci
- facultativement de 0,1 % à 30 % en poids d'un acide gras comprenant de 6 à 24 atomes de carbone ;
- éventuellement de l'eau,
la teneur en eau et/ou la teneur du mélange de polyols étant adaptée pour garantir une viscosité inférieure à 10 000 cps (10 000 mPa.s), avantageusement inférieure à 5 000 cps (5 000 mPa.s) à une température de 20 °C, avantageusement à température comprise entre 10° et 20° C.

2. Mélange de monoalkanolamoide selon la revendication 1, comprenant de 17 à 70 % en poids d'eau et d'un mélange de polyols comprenant au moins un glycérol et au moins un ou plusieurs alkylèneglycols, avec une masse moléculaire comprise entre 62 g et 1 000 g, le rapport pondéral entre le glycérol et les alkylèneglycols présents dans le mélange de polyols étant compris entre 1:50 et 50:1, avantageusement entre 1:50 et 20:1.

3. Mélange de monoalkanolamide selon la revendication 1 ou 2, qui comprend de 30 % à 60 % en poids d'eau et de 5 % en poids jusqu'à 30 % en poids d'un tensioactif choisi parmi les tensioactifs amphotères ou zwitterioniques, les tensioactifs anioniques et les mélanges de ceux-ci.

4. Mélange de monoalkanolamide selon l'une quelconque des revendications précédentes, possédant une teneur en matières solides de 35 % à 40 % en poids, tout en possédant une viscosité inférieure à 10 000 cps (10 000 mPa.s), avantageusement, inférieure à 5 000 cps (5 000 mPa.s) à une température de 20 ° C, avantageusement à une température comprise entre 10 °C et 20 °C.

5. Mélange de monoalkanolamide selon l'une quelconque des revendications précédentes, comprenant :
- de 17 à 35 % en poids, avantageusement de 20 % à 30 % en poids d'un mélange de polyols comprenant au moins un glycérol et au moins un alkylèneglycol avec une masse moléculaire comprise entre 62 g et 1 000 g, le rapport pondéral entre le glycérol et les. alkylèneglycols présents dans le mélange de polyols étant compris entre 1:50 et 50:1, avantageusement entre 1:50 et 20:1.

6. Mélange de monoalkanolamide selon l'une quelconque des revendications précédentes, le rapport pondéral entre le monoalkanolamide de formule (I) et le mélange de polyols étant compris entre 0,5 et 2.

7. Mélange de. monoalkanolamide selon l'une quelconque des revendications précédentes, dans lequel au moins 90 % en poids, avantageusement au moins 95 % en poids, préférablement au moins 97 % en poids du mélange est constitué du monoalkanolamide de formule (I), du mélange de polyols, du tensio-actif, d'eau et d'alcanolamine.

8. Procédé de mélange à froid destiné à la préparation d'un nettoyant liquide aqueux contenant du monoalkanolamide, ledit procédé comprenant l'étape de mélange à froid d'un mélange de monoalkanolamide selon l'une quelconque des revendications précédentes en une formulation aqueuse préparée séparément comprenant au moins un tensioactif ou l'étape de mélange à froid d'un composé supplémentaire en un mélange de monoalkanolamide selon l'une quelconque des revendications ci-dessus pour préparer ledit nettoyant liquide aqueux, ledit mélange de monoalkanolamide conférant au moins un comportement d'épaississement au nettoyant liquide aqueux.

9. Procédé de mélange pour la préparation d'un mélange selon l'une quelconque des revendications 1 à 7, ledit procédé comprenant au moins l'étape consistant à :
- chauffer (a) un mélange de polyols comprenant du glycérol et au moins un ou plusieurs alkylèneglycols avec un poids moléculaire compris entre 62 g et 1 000 g, jusqu'à une température comprise entre 40 °C et 90 °C, avantageusement entre 70 °C et 80 °C,
- facultativement, ajouter un tensioactif ou plus, de l'eau et/ou un acide gras ou plus ;
- ajouter le monoalkanolamide sous forme solide au mélange de polyols ayant une température comprise entre 40 °C et 90 °C,
- agiter le mélange pendant un temps suffisant, tout en maintenant la température dudit mélange à un niveau compris entre 40 °C et 90 C pour faire fondre pratiquement tout le monoalkanolamide présent dans le mélange,
- facultativement ajouter un ou plusieurs composants,
- refroidir le mélange contenant le monoalkanolamide à une température inférieure à 40 °C, tout en agitant,
- facultativement ajouter un ou plusieurs composants, et
- refroidir le mélange contenant le monoalkanolamide à une température inférieure à 30 °C, tout en agitant.

10. Procédé selon la revendication précédente, comprenant en outre au moins une étape de filtrage après l'étape de fusion de pratiquement tout le monoalkanolamide présent dans mélange contenant le polyol.

11. Procédé selon la revendication précédente, dans lequel le mélange contenant le monoalkanolamide contenant le mélange est filtré, tout en ayant une température comprise entre 40 °C et 90 °C.
